# EUROPÄISCHE PATENTANMELDUNG

(11) **EP 1 782 735 A2**
(43) Veröffentlichungstag der Anmeldung: **09.05.2007**
(21) Anmeldenummer: 06022849.1
(22) Anmeldetag: 02.11.2006
(51) Int. Cl.: A61B 6/00, A61B 19/00

(54) **Fahrgestell für eine mobile Röntgendiagnostikeinrichtung**

(30) Priorität: 05.11.2005 DE 102005052786
(71) Anmelder: Ziehm Imaging GmbH, 90451 Nürnberg (DE)
(72) Erfinder: Dehler, Jürgen, 91301 Forchheim (DE); Burgkart, Rainer, Dr., 80469 München (DE)

(57) **Zusammenfassung**

Die Anmeldung betrifft ein Fahrgestell (1) für eine mobile Röntgendiagnostikeinrichtung mit einem Lageerfassungssystem (12), das Meßeinrichtungen zur Bestimmung der Position des Fahrgestells in einem raumfesten Koordinatensystem enthält. Es ist vorgesehen, dass die Meßeinrichtungen auf berührungslosen Meßverfahren beruhen und die Position des Fahrgestells durch Triangulation ermittelt wird. Eine Röntgendiagnostikeinrichtung mit einem derartigen Lageerfassungssystem bietet Vorteile bei der 3D-Rekonstruktion aus 2D-Röntgenprojektionsaufnahmen und bei navigationsgeführten Eingriffen unter Röntgenkontrolle.

## Beschreibung

Die Erfindung betrifft ein Fahrgestell für eine mobile Röntgendiagnostikeinrichtung mit einem an diesem angeordneten Lageerfassungssystem.

Aus der US-Patentschrift US6374937B1 ist ein Fahrgestell für ein mobiles C-Bogen-Röntgengerät bekannt, das ein automatisches Verfahren der Röntgendiagnostikeinrichtung im wesentlichen senkrecht zu der Ebene des C-Bogens zum Zweck der Aufnahme einer Reihe von Röntgenaufnahmen. Ein Meßsystem erfaßt den von dem Fahrgestell zurück gelegten Weg.

Aus der deutschen Patentschrift DE4423359B4 der Anmelderin ist eine mobile Röntgendiagnostikeinrichtung mit motorisch angetriebenen und mit Inkrementalgebern ausgerüsteten Rollen bekannt, die eine motorische Verschiebung des C-Bogens längs des Fußbodens erlaubt. Die Lageerfassung beschränkt sich auf die relative Position des Fahrgestelles längs eines geradlinigen Verfahrweges.

Der Wunsch der Betreiber heutiger Röntgendiagnostikeinrichtungen geht dahin, mobile Röntgendiagnostikeinrichtungen zusammen mit Instrumentennavigationssystemen zur Unterstützung von computergestützten chirurgischen Eingriffen (CAS) oder von Kathederdiagnostik und -therapie einzusetzen. Hierfür wird mittels aus einer Anzahl von 2D-Röntgenprojektionsaufnahmen ein 3D-Volumenmodell rekonstruiert in dem beispielsweise ein Instrument navigiert werden kann. Die Güte der 3D-Rekonstruktion ist von der genauen Kenntnis der Projektionsgeometrie bei jeder 2D-Projektionsaufnahme abhängig. Bedingt durch die gegenüber stationären Anlagen leichtere Bauweise einer mobilen Röntgendiagnostikeinrichtung weichen die tatsächlichen Röntgenprojektionsgeometrien von den theoretisch aus der Kinematik errechneten Werten ab. Um hier Abhilfe zu schaffen, werden diese Abweichungen in einem Kalibrierlauf bestimmt und in Korrekturtabellen hinterlegt. Dieses Verfahren bietet eine wesentliche Verbesserung der Güte der 3D-Rekonstruktion für den Fall, daß die Fußbodenbeschaffenheit während des Kalibriervorganges, der meist im Herstellerwerk stattfindet, und während der Operation im Operationsraum identisch sind. Dies ist jedoch sehr häufig nicht der Fall und so entstehen zusätzliche Abweichungen, die in einem Kalibrierlauf nicht ermittelt werden können. Die Abweichungen können beispielsweise durch ein Eindrücken der Rollen des Fahrgestells in einen elastischen Fußbodenbelag, durch eine temporäre oder bleibende Abplattung der Laufflächen der Rollen aber auch durch die räumlich unterschiedliche Elastizität des Fußbodens, wie sie beispielsweise bei Hohlfußböden oder bei Containerfußböden mit partiellen Abstützungen auftritt, bewirkt werden.

Partielle Unterschiede in der Elastizität oder/und in der Neigung des Fußbodens wirken sich auch dann störend aus, wenn beispielsweise der Weg einer Kathederspitze in einem Untersuchungsobjekt verfolgt werden soll und der zu beobachtende Weg eine größere Länge als die größte Abmessung des Röntgenstrahlenempfängers aufweist. In diesem Fall muß eine Röntgenprojektion des Weges aus mehreren kleineren Projektionsaufnahmen zusammen gesetzt werden, wobei die Röntgendiagnostikeinrichtung in aller Regel auf dem Fußboden verfahren werden muß. Gleiches gilt für den Fall, daß von einem größeren, länglichen Untersuchungsbereich ein Volumenmodell aus 2D-Projektionsaufnahmen erzeugt werden soll.

Eine mobile Röntgendiagnostikeinrichtung wie sie aus der DE4423359B4 bekannt ist, kann in Verbindung mit einem Navigationssystem zur Instrumentennavigation in einem rekonstruierten 3D-Röntgenvolumen herangezogen werden, wenn die Position des.rekonstruierten Röntgenvolumens in Bezug auf das Koordinatensystem des Navigationssystems bekannt ist. Hierfür sind die in der DE4423359B4 vorgesehenen Meßmittel in der Praxis nicht ausreichend, da Abweichungen des Fußbodens von der Horizontalen und/oder lokale Schwankungen der Elastizität die Positionsbestimmung des rekonstruierten Röntgenvolumens verfälschen.

Aufgabe der Erfindung ist es, eine mobile Röntgendiagnostikeinrichtung zu schaffen, die Bestimmung der Orientierung des Wagens in einem raumfesten Koordinatensystem auch dann erlaubt, wenn der Fußboden uneben ist oder/und lokale Unterschiede in der Elastizität aufweist.

Die Aufgabe der Erfindung wird dadurch gelöst, daß die Röntgendiagnostikeinrichtung auf einem Wagen angeordnet ist, der ein mit einem Referenzkörper ausgestattetes Lageerfassungssystem trägt, das die Lage des Referenzkörpers im Raum bestimmt und an eine Steuer der Röntgendiagnostikeinrichtung zur Bestimmung der 2D-Projektionsgeometrie übermittelt.

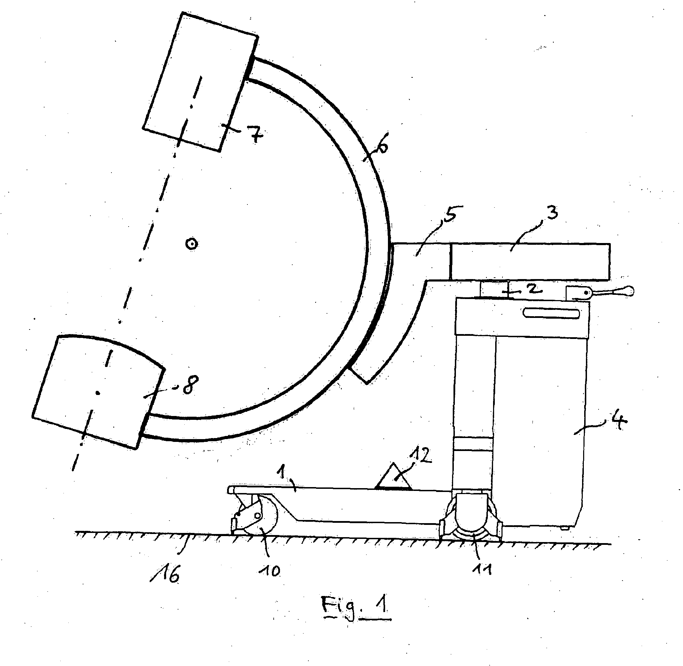

Die Erfindung wird nachstehend an Hand der Fig. 1 näher erläutert.

An einem annähernd kreisbogenförmigen C-Bogen 6 sind gegenüberliegend am einen Ende eine Röntgenstrahlenquelle 8 und am anderen Ende ein Röntgenstrahlenempfänger 7 angeordnet. Der C-Bogen 6 ist längs seines Umfangs in einer Halterung 5 verschieblich gelagert, mit einem Positionssensor für die Verschiebebewegung und vorzugsweise mit einem elektromotorischen Antrieb ausgestattet. Die Halterung 5 ist an einer ebenfalls vorzugsweise elektromotorisch angetriebenen und mit einem Positionssensor versehenen Horizontalführung 3 angeordnet, welche an einer vertikalen Säule 2 elektromotorisch höhenverstellbar gelagert ist. Die Führung der Säule 2 ist starr mit einem Fahrgestell 1 verbunden, der auch den Geräteschrank 4 trägt.

Das Fahrgestell 1 weist lenkbare und vorzugsweise motorisch angetriebene Rollen 10, 11 auf, die auf dem Fußboden 16 verfahrbar sind.

An dem Fahrgestell 1 ist ein Lageerfassungssystem 12 derart angeordnet, daß die Orientierung eines mit dem Lageerfassungssystem 12 verbundenen Referenzkörpers im Raum bestimmbar ist. Das Lageerfassungssystem kann eine berührungslos arbeitende Anordnung von Abstandssensoren aufweisen, mittels derer beispielsweise raumfeste Markierungen erkannt und deren Abstand zu dem Lageerfassungssystem 12 bestimmt wird. Die Orientierung des Referenzkörpers wird trivial durch Anwenden eines bekannten Triangulationsverfahrens ermittelt.

Im Rahmen der Erfindung ist die Verwendung eines Lageerfassungssystems 12 vorgesehen, das Mittel zur Bestimmung der Neigung einer Referenzfläche am Fahrgestell und ein Wegmeßsystem zur Bestimmung der Position des Fahrgestells 1 bezüglich des Fußbodens enthält. Bei einem solchen Wegmeßsystem kann es sich um eine Anordnung von auf dem Fußboden abrollenden Meßkugeln oder um berührungslos arbeitende Wegmeßsysteme, z.B. Laser- oder LED-Meßsysteme handeln. Es ist ferner vorgesehen, für das Wegmeßsystem magnetische oder elektromagnetische Sensoren einzusetzen, die mit damit sensierbaren Führungsdrähten oder Strukturen im Fußboden zusammen wirken. Das Wegmeßsystem ist in der Lage, die Koordinaten des Fahrgestells 1 relativ zu einem fußbodenfesten Koordinatensystem beim Beginn der Translationsbewegung zu bestimmen. Dafür ist beispielsweise vorgesehen, eine für das Wegmeßsystem erkennbare Markierung auf dem Fußboden anzubringen, die den Urspung und die Achsen des fußboden- bzw. raumfesten Koordinatensystems festlegt, auf das sich die Translationen des' Fahrgestells 1 beziehen.

### Bezugszeichenliste:

- 1: Fahrgestell
- 2.: Säule
- 3: Horizontalführung
- 4: Geräteschrank
- 5: Halterung
- 6: C-Bogen
- 7: Röntgenstrahlenempfänger
- 8: Röntgenstrahlenquelle
- 10: Rolle
- 11: Rolle
- 12: Lageerfassungssystem
- 16: Fußboden

## Patentansprüche

1. Mobile Röntgendiagnostikeinrichtung zur Erzeugung von 2D-Röntgenprojektionsaufnahmen für eine Volumenrekonstruktion mit einer an einem mehrfach motorisch verstellbaren C-Bogen angeordneten Röntgenstrahlenquellen-/Röngtenstrahlenempfänger-Einheit und einem längs des Fußbodens 16 verfahrbaren Fahrgestells 1,
**dadurch gekennzeichnet, daß** an einem Referenzkörper des Fahrgestells 1 ein Lageerfassungssystem 12 angeordnet ist, das die Position des Referenzkörpers in einem raumfesten Koordinatensystem ermittelt und die Position des Referenzkörpers an einen Steuer- und Bildverarbeitungsrechner der Röntgendiagnostikeinrichtung zur Berechnung der jeweils aktuellen Röntgenprojektionsgeometrie übermittelt.

2. Mobile Röntgendiagnostikeinrichtung nach Anspruch 1,
**dadurch gekennzeichnet, daß** das Lageerfassungssystem 12 LASER-Abstandsmeßaufnehmer enthält, die die Abstände des Lageerfassungssystems zu raumfesten Markierungen ermitteln.

3. Mobile Röntgendiagnostikeinrichtung nach Anspruch 1,
**dadurch gekennzeichnet, daß** das Lageerfassungssystem 12 einen Neigungssensor zur Ermittlung der Neigung einer Referenzfläche des Fahrgestells 1 gegenüber der Richtung der Erdbeschleunigung und wenigstens drei mechanisch mit dem Fußboden verbundene Meßräder aufweist deren Rotation von Inkrementalgebern detektiert wird.

4. Mobile Röntgendiagnostikeinrichtung nach Anspruch 1,
**dadurch gekennzeichnet, daß** das Lageerfassungssystem 12 einen Neigungssensor zur Ermittlung der Neigung einer Referenzfläche des Fahrgestells 1 gegenüber der Richtung der Erdbeschleunigung und berührungslos arbeitende optische Wegmeßaufnehmer aufweist, die die relative Translation des Fahrgestells 1 gegenüber dem Fußboden 16 ermitteln.

5. Mobile Röntgendiagnostikeinrichtung nach Anspruch 4,
**dadurch gekennzeichnet, daß** das Lageerfassungssystem 12 Wegmeßaufnehmer aufweist, die eine periodische elektrische Struktur im Fußboden detektieren.

6. Mobile Röntgendiagnostikeinrichtung nach Anspruch 4,
**dadurch gekennzeichnet, daß** das Lageerfassungssystem 12 Wegmeßaufnehmer aufweist, die eine periodische magnetische Struktur im Fußboden detektieren
